# EUROPÄISCHE PATENTSCHRIFT

(11) **EP 1 648 565 B1**
(45) Veröffentlichungstag und Bekanntmachung des Hinweises auf die Patenterteilung: **01.04.2009**
(21) Anmeldenummer: 04740326.6
(22) Anmeldetag: 25.06.2004
(51) Int. Cl.: A61P 5/50, A61K 36/18

(54) **ARZNEIMITTEL UMFASSEND PFLANZENMATERIAL VON MASCAGNIA EGGERSIANA, ZUR BEHANDLUNG VON DIABETES MELLITUS TYP 2**
MEDICAMENT COMPRISING PLANT MATERIAL FROM MASCAGNIA EGGERSIANA FOR TREATING TYPE 2 DIABETES MELLITUS
MEDICAMENT A BASE DE MATIERE VEGETALE DE MASCAGNIA EGGERSIANA, UTILISE DANS LE TRAITEMENT DU DIABETE SUCRE DE TYPE II

(30) Priorität: 25.06.2003 DE 10328597
(43) Veröffentlichungstag der Anmeldung: 26.04.2006
(73) Patentinhaber: Glaser, Roland, Quito (EC); Huber, Christian, 81539 München (DE)
(72) Erfinder: Glaser, Roland, Quito (EC)
(74) Vertreter: Zimmermann & Partner
(86) Internationale Anmeldenummer: PCT/EP2004/006917
(87) Internationale Veröffentlichungsnummer: WO 2004/112904

(56) Entgegenhaltungen:
- ANONYMOUS: "Ayahuasca recipes" INTERNET ARTICLE, [Online] 27. April 2003 (2003-04-27), XP002300492 Gefunden im Internet: <URL:www.biopark.org/ayarecipe.html> [gefunden am 2004-10-12]
- DATABASE BIOSIS [Online] BIOSCIENCES INFORMATION SERVICE, PHILADELPHIA, PA, US; April 1998 (1998-04) TOKARNIA CARLOS HUBINGER ET AL: "Experimental poisoning in rabbits by, Mascagnia sp (Malpighiaceae) collected in the State of Santa Catarina, southern Brazil" Database accession no. PREV199800489138 XP002300494
- ARTSCHWAGER KAY M.: "Healing with Plants in the American and Mexican West" INTERNET ARTICLE, [Online] 1996, XP002300493 Gefunden im Internet: <URL:www.uapress.arizona.edu/books/bid977. htm> [gefunden am 2004-10-12]

## Beschreibung

Die vorliegende Erfindung betrifft ein Arzneimittel, welches Pflanzenmaterial bzw. pflanzliche Wirkstoffe enthält. Insbesondere betrifft die vorliegende Erfindung ein Arzneimittel zur Behandlung und/oder Prophylaxe von Diabetes mellitus Typ 2, sowie ein Arzneimittel zur Verbesserung der Durchblutung, insbesondere von peripheren Blutgefäßen.

Diabetes mellitus Typ 2, auch Altersdiabetes genannt, ist eine Erkrankung des Kohlehydratstoffwechsels, die typischerweise ab dem 30. Lebensjahr einsetzt. In der jüngeren Vergangenheit wurde jedoch beobachtet, dass diese Erkrankung zunehmend auch bei jungen übergewichtigen Menschen auftritt. Nach Schätzungen der WHO waren im Jahr 1998 mehr als 143 Millionen Menschen an Diabetes mellitus Typ 2 erkrankt, bis zum Jahr 2025 wird eine Zunahme der Zahl an Erkrankten auf 300 Millionen erwartet.

Im Gegensatz zu Diabetes mellitus Typ 1, der eine reine Insulin-Mangelkrankheit ist, ist die Entstehung von Diabetes mellitus Typ 2 nicht auf einen Insulinmangel zurückzuführen. Vielmehr handelt es sich bei Diabetes mellitus Typ 2 um eine verminderte Insulinwirkung, bei welcher der Körper auf dieses Hormon nicht mehr richtig anspricht. Es wird davon ausgegangen, dass die Ursachen dafür in einem Defekt der Körperzellen liegen, Insulin in ausreichender Menge aufzunehmen, oder/und in einem Defekt der Langerhans'schen Zellen der Bauchspeicheldrüse, Insulin bedarfsgerecht bereitzustellen. Dieser Zustand wird allgemein als Insulinresistenz bezeichnet. Der Körper versucht im Anfangsstadium der Erkrankung das Gleichgewicht durch erhöhte Insulinproduktion wieder herzustellen, was zu einer immer höheren Insulinproduktion führt und in späteren Stadien der Erkrankung zu einer Erschöpfung der Bauchspeicheldrüse führen kann.

Diabetes mellitus Typ 2 wird unterschieden in Typ 2A mit normalem Körpergewicht, Typ 2B mit Übergewicht, sowie dem medikamentös induzierten Typ 2C. Am häufigsten ist in den Industrieländern der mit Übergewicht assoziierte Diabetes Typ 2B, weshalb die Erkrankung häufig zu den so genannten Wohlstandserkrankungen gerechnet wird.

Die Behandlung von Diabetes mellitus Typ 2 umfasst primär eine Gewichtsreduktion auf Basis einer ausgewogenen Diät zusammen mit regelmäßiger körperlicher Betätigung. Wenn diese Allgemeinmaßnahmen nicht zum gewünschten Erfolg führen, kann eine Behandlung mit Arzneimitteln erforderlich werden.

Als Arzneimittel zur Behandlung von Diabetes mellitus Typ 2 können die folgenden Gruppen unterschieden werden:
- Resorptionverzögerer wie etwa Acarbose verzögern die Aufnahme von Glucose aus dem Darm und vermeiden somit Blutzucker-Spitzen,
- Biguanide wie etwa Metformin hemmen die Gluconeogenese und verringern die Insulinresistenz,
- Sulfonylharnstoffe wie etwa Glibenclamid regen die Insulinproduktion an,
- Insulin-Sensitizer wie etwa Glitazone verbessern die Insulinsensitivität der Zellen, und
- Insulin- und Insulin-Analoga.

Es besteht jedoch weiterhin ein Bedarf an neuen Arzneimitteln zur Behandlung oder/und Prophylaxe von Diabetes mellitus Typ 2. Derartige Arzneimittel sollten für den Patienten gut verträglich sein, und so wenig schädliche Nebenwirkungen wie möglich aufweisen.

Gegenstand der vorliegenden Erfindung ist somit, ein Arzneimittel zur Behandlung oder/und Prophylaxe von Diabetes mellitus Typ 2 bereitzustellen.

Es wurde nunmehr überraschenderweise festgestellt, dass
Mascagnia eggersiana (Nied.) W.R. Anderson
(nachfolgend abgekürzt als Mascagnia eggersiana bezeichnet) zur Behandlung von Diabetes mellitus Typ 2 geeignet ist.

Mascagnia eggersiana ist eine Pflanze aus der Familie der Malphigiaceae mit einem gesicherten Vorkommen in der Provinz Napo in Ecuador und einem Verbreitungsgebiet, das sich zumindest entlang des Andenosthangs von Ecuador ab einer Höhe von ca. 500-600 m und darunter erstreckt. Über eine weitere Ausdehnung des Verbreitungsgebiets liegen derzeit keine näheren Informationen vor. Unter der Bezeichnung Mascagnia eggersiana werden in der vorliegenden Anmeldung sowohl die bezeichnete Art als auch Ökotypen und Unterarten davon verstanden. Die Begriffe "Ökotyp" und "Unterart" werden hierin im üblichen Sinne verwendet.

Ein Referenzbeleg von Mascagnia eggersiana findet sich in Deutschland mindestens in der Botanischen Staatssammlung München, im außereuropäischen Ausland finden sich Referenzbelege zumindest in mehreren botanischen Instituten innerhalb der USA. Am botanischen Institut der Universität München wird derzeit weiterhin ein Exemplar von Mascagnia eggersiana in Kultur gehalten.

Gegenstand der vorliegenden Erfindung ist somit ein Arzneimittel, umfassend Pflanzenmaterial von Mascagnia eggersiana, Unterarten, Ökotypen oder einer Kombination davon. Ein weiterer Gegenstand der vorliegenden Erfindung ist ein Arzneimittel, umfassend pflanzliche Wirkstoffe, dadurch gekennzeichnet, dass die Wirkstoffe aus Pflanzen, ausgewählt aus Mascagnia eggersiana, Unterarten, Ökotypen oder einer Kombination davon, erhältlich sind.

Gemäß einem ersten Aspekt ist das erfindungsgemäße Arzneimittel wie vorstehend beschrieben ein Arzneimittel zur Prophylaxe oder Behandlung von Diabetes mellitus Typ 2. Bei den durchgeführten Tests hat sich jedoch darüber hinaus herausgestellt, dass die Testpersonen nach wenigen Tagen ein "Kribbeln" in den peripheren Gliedmaßen wie etwa Zehen und Fingerspitzen verspürten, und die Durchblutung wieder in Gang geriet. Gemäß einem zweiten Aspekt ist das erfindungsgemäße Arzneimittel somit ein Arzneimittel zur Verbesserung der Durchblutung, insbesondere von peripheren Blutgefäßen.

Gemäß einer bevorzugten Ausführungsform des die Wirkstoffe umfassenden Arzneimittels werden die Wirkstoffe aus Mascagnia eggersiana, Unterarten, Ökotypen oder einer Kombination davon erhalten.

Die bisherigen Versuche wurden vorwiegend mit Jungpflanzen durchgeführt, die als Stecklinge vermehrt worden waren und die Morphologie eines Busches mit einem Durchmesser von etwa 0,3 bis 1,2 m hatten. In späteren Entwicklungsstadien bildet Mascagnia eggersiana Fortsätze mit einer Höhe von mehreren Metern. Somit umfasst das Arzneimittel in einer besonderen Ausführungsform Pflanzenmaterial bzw. Wirkstoffe von Mascagnia eggersiana, Unterarten, Ökotypen oder einer Kombination davon, die von Jungpflanzen stammen, beispielsweise von Jungpflanzen mit einer überirdischen Höhe von kleiner 1,2 m, insbesondere 0,3 bis 1 m.

Gemäß einer besonderen Ausführungsform stammt das Pflanzenmaterial aus der Wurzel, bzw. werden die Wirkstoffe aus der Wurzel erhalten. Andere Teile der Pflanze, wie etwa der Pflanzenkörper oder die Blätter sind jedoch ebenfalls geeignet.

Beispielsweise kann die Wurzel als solche gegessen, d.h. oral eingenommen werden, oder getrocknet werden und, zweckmäßig zu einem Pulver vermahlen, oral eingenommen werden. Zur Trocknung der Wurzeln ist eine einfache Trocknung an Luft bei Raumtemperatur ausreichend und geeignet. Vor dem Verzehr bzw. dem Vermahlen werden zweckmäßig holzartige Teile wie etwa der holzige Stiel der Wurzel entfernt.

Ein nochmals weiterer Gegenstand der vorliegenden Erfindung ist die Verwendung von Pflanzenmaterial von Mascagnia eggersiana, Unterarten, Ökotypen oder einer Kombination davon, zur Herstellung eines Arzneimittels zur Prophylaxe oder Behandlung von Diabetes mellitus Typ 2 bzw. zur Verbesserung der Durchblutung, insbesondere von peripheren Blutgefäßen.

Ein nochmals weiterer Gegenstand der vorliegenden Erfindung ist die Verwendung von pflanzlichen Wirkstoffen, erhältlich aus Pflanzen, ausgewählt aus Mascagnia eggersiana, Unterarten, Ökotypen oder einer Kombination davon, zur Herstellung eines Arzneimittels zur Prophylaxe oder Behandlung von Diabetes mellitus Typ 2, bzw. zur Herstellung eines Arzneimittels zur Verbesserung der Durchblutung, insbesondere von peripheren Blutgefäßen.

Besondere und bevorzugte Ausführungsformen der erfindungsgemäßen Verwendungen sind wie vorstehend in Zusammenhang mit den erfindungsgemäßen Arzneimitteln beschrieben.

Gemäß einer besonderen Ausführungsform der betreffenden erfindungsgemäßen Verfahren werden die Wirkstoffe aus Mascagnia eggersiana, Unterarten, Ökotypen oder einer Kombination davon erhalten.

Die in den erfindungsgemäßen Verfahren zu verabreichende Dosis hängt vom Erkrankungszustand des Patienten, sowie von seinem Alter, Gewicht und physischen Allgemeinzustand ab. Bei den bisherigen Versuchen wurde eine Dosis von dreimal täglich einem gestrichenen Esslöffel verwendet, die morgens, mittags und abends verabreicht wurden. Nebenwirkungen wurden bei dieser Dosismenge bisher nicht beobachtet. Gemäß einer bevorzugten Ausführungsform beträgt die Gesamttagesdosis 1-100 g, bezogen auf getrocknetes Pflanzenmaterial, insbesondere 10-80 g, beispielsweise 25-50 g.

## Patentansprüche

1. Arzneimittels, umfassend Pflanzenmaterial von Mascagnia eggersiana, Unterarten, Ökotypen oder einer Kombination davon.

2. Arzneimittel nach Anspruch 1, zur Prophylaxe oder Behandlung von Diabetes mellitus Typ 2.

3. Arzneimittel nach Anspruch 1, zur Verbesserung der Durchblutung, insbesondere von peripheren Blutgefäßen.

4. Arzneimittel nach einem der Ansprüche 1 bis 3, umfassend Pflanzenmaterial von Jungpflanzen.

5. Arzneimittel nach einem der vorhergehenden Ansprüche, **dadurch gekennzeichnet, dass** das Pflanzenmaterial aus der Wurzel stammt.

6. Verwendung von Pflanzenmaterial von Mascagnia eggersiana, Unterarten, Ökotypen oder einer Kombination davon, zur Herstellung eines Arzneimittels zur Prophylaxe oder Behandlung von Diabetes mellitus Typ 2.

7. Verwendung von Pflanzenmaterial von Mascagnia eggersiana, Unterarten, Ökotypen oder einer Kombination davon, zur Herstellung eines Arzneimittels zur Verbesserung der Durchblutung, insbesondere von peripheren Blutgefäßen.

8. Verwendung nach einem der Ansprüche 6 oder 7, wobei man Jungpflanzen verwendet.

9. Verwendung nach einem der Ansprüche 6 bis 8, wobei man die Wurzel verwendet.

## Claims

1. A medicament, comprising plant material of Mascagnia eggersiana, subspecies, ecotypes or a combination thereof.

2. The medicament of claim 1, for the prophylaxis or treatment of diabetes mellitus type 2.

3. The medicament of claim 1, for improving the blood flow, in particular in peripheral blood vessels.

4. The medicament of one of claims 1 to 3, comprising plant material derived from young plants.

5. The medicament of one of the preceding claims, **characterized in that** said plant material is derived from the root.

6. Use of plant material of Mascagnia eggersiana, subspecies, ecotypes or a combination thereof for the preparation of a medicament for the prophylaxis or treatment of diabetes mellitus type 2.

7. Use of plant material of Mascagnia eggersiana, subspecies, ecotypes or a combination thereof for the preparation of a medicament for improving the blood flow, in particular in peripheral blood vessels.

8. The use of one of claims 6 or 7, wherein young plants are used.

9. The use of one of claims 6 to 8, wherein the root is used.

## Revendications

1. Médicament, comprenant de matière végétale de Mascagnia eggersiana, sous-espèces, écotypes ou leur combinaison.

2. Médicament selon la revendication 1, pour la prophylaxie ou le traitement de diabète sucré de type 2.

3. Médicament selon la revendication 1, pour la amélioration de la circulation du sang, en particulier de vaissaux sanguins périfériques.

4. Médicament selon une des revendications 1 à 3, comprenant matière végétale de plantes jeunes.

5. Médicament selon une des revendications précédentes, **caractérisé en ce que** la matière végétale est derivée de la racine.

6. Utilisation de matière végétale de Mascagnia eggersiana, sous-espèces, écotypes ou leur combinaison pour la préparation d'un médicament pour la prophylaxie ou le traitement de diabète sucré de type 2.

7. Utilisation de matière végétale de Mascagnia eggersiana, sous-espèces, écotypes ou leur combinaison pour la préparation d'un médicament pour la amélioration de la circulation du sang, en particulier de vaissaux sanguins périfériques.

8. Médicament selon une des revendications 6 ou 7, où on utilise de plantes jeunes.

9. Médicament selon une des revendications 1 à 8, où on utilise la racine.
